(19) Europäisches Patentamt
European Patent Office
Office européen des brevets

(11) EP 4 534 083 A1

# (12) EUROPEAN PATENT APPLICATION
published in accordance with Art. 153(4) EPC

(43) Date of publication:
**09.04.2025 Bulletin 2025/15**

(21) Application number: **23815144.3**

(22) Date of filing: **29.05.2023**

(51) International Patent Classification (IPC):
***A61K 31/4375*** *(2006.01)* ***A61P 41/00*** *(2006.01)*
***A61P 1/00*** *(2006.01)*

(52) Cooperative Patent Classification (CPC):
**A61K 31/4375; A61P 1/00; A61P 41/00**

(86) International application number:
**PCT/CN2023/096840**

(87) International publication number:
**WO 2023/231964 (07.12.2023 Gazette 2023/49)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **30.05.2022 CN 202210596510**
**20.10.2022 CN 202211286168**

(71) Applicant: **Scinnohub Pharmaceutical Co., Ltd.**
**Chengdu, Sichuan 610094 (CN)**

(72) Inventors:
- **HU, Xiao**
**Chengdu, Sichuan 610094 (CN)**
- **WANG, Yan**
**Chengdu, Sichuan 610094 (CN)**
- **JI, Sen**
**Chengdu, Sichuan 610094 (CN)**
- **WANG, Xiao**
**Chengdu, Sichuan 610094 (CN)**
- **ZHANG, Xiaodong**
**Chengdu, Sichuan 610094 (CN)**
- **TANG, Jun**
**Chengdu, Sichuan 610094 (CN)**

(74) Representative: **Gille Hrabal Partnerschaftsgesellschaft mbB**
**Patentanwälte**
**Brucknerstraße 20**
**40593 Düsseldorf (DE)**

## (54) USE OF TETRAHYDRONAPHTHYRIDINE DERIVATIVE FOR PREPARATION OF MEDICINE FOR PREVENTING AND TREATING ADHESION-RELATED DISEASES

(57) Provided is use of a tetrahydronaphthyridine derivative in preparing a medicine for preventing and treating gastrointestinal tract injury-related diseases or inhibiting gastrointestinal protein kinase; the compound achieves a good effect of preventing and treating the gastrointestinal tract injury-related diseases.

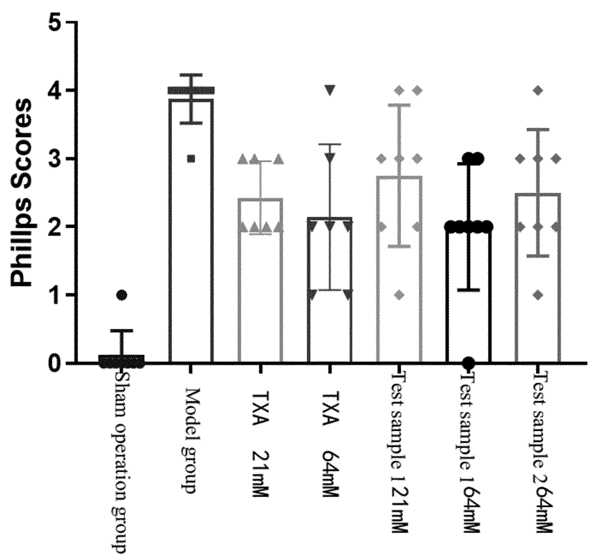


FIG. 4

EP 4 534 083 A1

## Description

### CROSS-REFERENCE TO RELATED APPLICATION

This application claims priority to Chinese patent application No. 202210596510.4 filed to State Intellectual Property Office on May 30, 2022 and entitled "Use of Tetrahydronaphthyridine Derivative in Preparing Medicine for Preventing and Treating Adhesion-related Diseases", and Chinese patent application No. 202211286168.4 filed to State Intellectual Property Office on October 20, 2022 and entitled "Use of Tetrahydronaphthyridine Derivative in Preparing Medicine for Preventing and Treating Adhesion-related Diseases", the entire contents of which are incorporated herein by reference.

### TECHNICAL FIELD

The present disclosure relates to the field of medicines, and in particular to use of a tetrahydronaphthyridine derivative in preparing a medicine for preventing and treating adhesion-related diseases.

### BACKGROUND

Adhesions are the result of tissue injury and the inducements include sharp objects, mechanical or thermal damages, infection, radiation, ischemia, dryness, abrasion, foreign body reaction, tumors, etc. Regardless of the triggering factors, adhesions are formed by the interaction of three intertwined processes in the body, i.e., the fibrinolytic system, the deposition and remodeling of the extracellular matrix, and the inflammatory system. When the gastrointestinal tract is subjected to biological, physical, or chemical damages, an acute inflammatory reaction will occur. Tissue ischemia, tissue factors, and cytokines, increased vascular permeability, and release of inflammatory factors, and the like trigger intestinal adhesions and intraperitoneal adhesions; moreover, a large amount of exudate containing fibrinogen and fibrin can be deposited and coagulated into a fibrous network, which is concentrated on the surface of the stimulated tissues and organs and adheres to the peripheral tissues. Under normal circumstances, in addition to releasing the fibrinogen, the exudate of peritoneal mesothelial cells also has the fibrinolytic effect, which can dissolve and absorb the cellulose network, thereby reducing the formation of adhesions. The balance between the deposition and degradation of fibrin plays a key role in normal repair or healing or adhesion formation. The key factor leading to the imbalance between fibrin deposition and degradation is local injury and the degree of inflammatory reaction.

Postoperative adhesions, such as intestinal adhesions or peritoneal adhesions, may cause infertility, pain, or intestinal obstruction and may increase the difficulty of subsequent abdominal or pelvic surgery. All surgeons shall be familiar with the risks and consequences of postoperative adhesions and take measures to minimize the occurrence of postoperative adhesions. Theoretically, the adhesion formation can be reduced by reducing peritoneal injury during surgery, preventing the introduction of reactive foreign bodies, reducing local inflammatory reactions, inhibiting the coagulation cascade reaction, and promoting fibrinolysis or setting up a barrier among damaged tissues. However, studies have shown that surgeries such as hysteromyomectomy often lead to adhesions regardless of whatever surgical methods are chosen. The incidence of adhesions after open myomectomy is greater than 90%, but it is still at least 70% after laparoscopic myomectomy. The use of anti-adhesion bioresorbable membranes to physically isolate the surgical site from the adjacent peritoneum or adjacent organs and prevent mutual adhesion is considered one of the feasible methods. The FDA has approved a variety of anti-adhesion film products, such as Seprafilm, a film composed of hyaluronic acid and carboxymethylcellulose. Clinical studies have shown that the postoperative bowel sound recovery time and time of intestinal exhaust in the patients in the experimental group with absorbable medical films placed on the wound surface and other parts after abdominal surgery are significantly shorter than those of the patients in the control group who have not undergone postoperative interventions, and their postoperative physical signs are superior to the patients in the control group, and the absorbable medical films feature good biocompatibility and safety. The bioresorbable films can effectively prevent postoperative adhesions, reduce the risk of secondary surgery, and improve compliance remarkably. However, the action site of the bioresorbable films is only limited to the placement site, and surgeons need to judge the possible adhesion site, and the adhesion site may have a certain distance from the surgical site; moreover, it is difficult to place the anti-adhesion membrane, so the bioresorbable films are hardly used in laparoscopic surgery.

Presently, there are many studies evaluating the efficacy of medicines in preventing adhesions, and these medicines include recombinant tPA, streptokinase, heparin, low molecular weight heparin, nonsteroidal anti-inflammatory agents, gonadotropin-releasing hormone agonists, phosphatidylcholine, vitamin E antioxidant molecules, and corticosteroids. These medicines are initially developed for the treatment of diseases other than postoperative adhesions. The mechanism of their prevention and treatment of adhesions is still unclear, and the effect still needs to be further verified. The latest studies have shown that intestinal trauma (incisions, surgical operations, and hypoperfusion) can lead to rupture of the intestinal mucosal barrier, and subsequently, digestive proteases are transferred into the intestinal tissues and

visceral cavity. These proteases can cause proteolytic damage to the mesothelial surface of the viscera, and as a part of the biological repair process, they can induce adhesion formation in the process of healing damaged tissues. The inventors have found in long-term studies that some specific tetrahydronaphthyridine derivatives can be used as broad-spectrum serine protease inhibitors to neutralize the activity of digestive proteases excessively released due to intestinal mucosal barrier damage during surgery, which reduces the damages to gastrointestinal tissues and accelerates the recovery of gastrointestinal functions, thereby achieving the purpose of preventing and treating postoperative intestinal adhesions.

## SUMMARY

An object of the present disclosure is to provide use of a tetrahydronaphthyridine derivative in preparing a medicine for preventing and treating gastrointestinal tract injury-related diseases.

In some specific embodiments, the tetrahydronaphthyridine derivative is selected from 5,6,7,8-tetrahydro-1,6-naphthyridine-2-carboxylic acid, 5,6,7,8-tetrahydro-1,6-naphthyridine-2-phosphonic acid, and a hydrate, or a pharmaceutically acceptable salt, or a prodrug, or a mixture thereof.

In some specific embodiments, the pharmaceutically acceptable salt is a hydrochloride of the tetrahydronaphthyridine derivative.

In some specific embodiments, the gastrointestinal tract injury-related diseases refer to adhesions, especially including various adhesions caused by abdominal gastrointestinal injury, infection, and the like after surgery, for example, postoperative intestinal adhesions, abdominal adhesions, intercostal adhesions, peritoneal adhesions, intrauterine adhesions and the like.

In some specific embodiments, the gastrointestinal tract injury-related diseases also include complications caused by the postoperative adhesions, such as one or more of female infertility, postoperative intestinal obstruction, and pain.

The present disclosure further provides use of a tetrahydronaphthyridine derivative in preparing a medicine for inhibiting gastrointestinal protein kinase.

In some specific embodiments, the gastrointestinal protein kinase is selected from at least one of Trypsin, MMP9, and TACE.

In the sense of the present disclosure, "prevention and treatment" refers to "prevention" and/or "treatment", where "prevention" means to avoid, or alleviate, or reduce the risk of the occurrence and development of the disease; "treatment" refers to eliminating, hindering, alleviating weakening, restricting, inhibiting or curbing the symptoms of diseases or the development, process or progression of the disease symptoms by means of artificial interventions.

### Beneficial effects

Studies in the present disclosure have shown that the tetrahydronaphthyridine derivative has a good effect of preventing and treating gastrointestinal tract injury-related diseases, can be used to prepare a medicine for preventing and treating gastrointestinal injury or inhibiting gastrointestinal protein kinase, and especially has a good preventive and therapeutic effect on diseases and complications such as gastrointestinal adhesions, abdominal adhesions, intercostal adhesions, peritoneal adhesions, and intrauterine adhesions.

## BRIEF DESCRIPTION OF DRAWINGS

FIG. 1 is photos of abdominal intestinal parts of rats executed on the 7$^{th}$ day after operation in a sham operation group in an Experimental Example 3;

FIG. 2 is photos of abdominal intestinal parts of rats executed on the 7$^{th}$ day after operation in a model group in the Experimental Example 3;

FIG. 3 is photos of abdominal intestinal parts of rats executed on the 7$^{th}$ day after operation in a medicine group in the Experimental Example 3;

FIG. 4 is a bar graph of scores of intestinal adhesions of rats in an Experimental Example 4 using the Phillips grading method.

## DETAILED DESCRIPTION

The technical solutions of the present disclosure will be further described in detail below in conjunction with specific examples. It should be understood that the following examples are only used for exemplary illustrations and

explanations of the present disclosure and should not be construed as limiting the scope of protection of the present disclosure. All technologies implemented based on the above content of the present disclosure are included in the scope of protection intended by the present disclosure.

Unless otherwise specified, the raw materials and reagents used in the following examples are commercially available products or can be prepared by known methods.

**Preparative example 1**

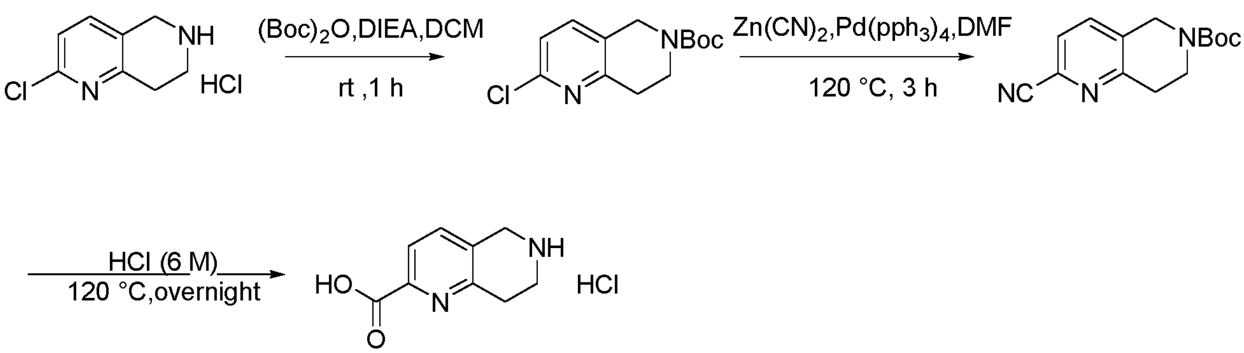


Step 1: Preparation of tert-butyl 2-chloro-7,8-dihydro-1,6-naphthyridine-6-(5H)-carboxylate

2-chloro-5,6,7,8-tetrahydro-1,6-naphthyridine hydrochloride (0.9 g) was weighed and suspended in 15 mL of dichloromethane, N,N-diisopropylethylamine (1.4 g) was added to dissociate, then di-tert-butyl dicarbonate (1.15 g) was added to react at room temperature for 1 hour. When the TLC showed the raw materials were completely consumed, purification was performed by column chromatography to obtain the title compound (1.12 g).

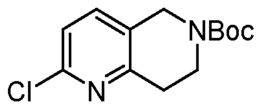

MS (ESI) m/z $(M+H)^+$=269.0.

Step 2: Preparation of tert-butyl 2-cyano-7,8-dihydro-1,6-naphthyridine-6-(5H)-carboxylate

tert-butyl 2-chloro-7,8-dihydro-1,6-naphthyridine-6(5H)-carboxylate (1.12 g) was weighed and dissolved in 20 mL of N,N-dimethylformamide, then zinc cyanide (2.44 g) and tetraki(triphenylphosphine) palladium (483 mg) were added, and argon replacement was performed for 3 times, and a reaction was carried out at 120°C for 3 hours. When the TLC showed the raw materials were completely consumed, ethyl acetate was added for dilution, and a mixture was filtered by diatomaceous earth and extracted with ethyl acetate twice, washed with saturated brine once and dried over anhydrous sodium sulfate, and subjected to purification by column chromatography to obtain the title compound (1.1 g).

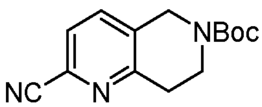

MS (ESI) m/z $(M+H)^+$=260.0.

Step 3: Preparation of 5,6,7,8-tetrahydro-1,6-naphthyridine-2-carboxylic acid hydrochloride

tertiary butyl 2-cyano-7,8-dihydro-1,6-naphthyridine-6-(5H)-carboxylate (1.1 g) was weighed and dissolved in 25 mL of a 6 M aqueous hydrochloric acid solution, and reacted at 120 °C overnight. When the LCMS showed that the raw materials were completely consumed, a reaction solution was concentrated to dryness, and separated by pre-HPLC to obtain the title compound (726 mg).

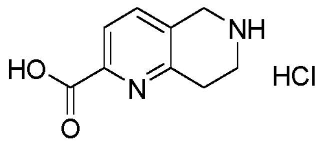

MS (ESI) m/z $(M+H)^+$=179.0.

$^1H$ NMR (400 MHz, Methanol-d4) δ 8.21 (d, *J* = 8.1 Hz, 1H), 8.12 (d, *J* = 8.0 Hz, 1H), 4.60 (s, 2H), 3.71 (t, *J* = 6.4 Hz, 2H), 3.42 (t, *J* = 6.4 Hz, 2H).

**Preparative example 2**

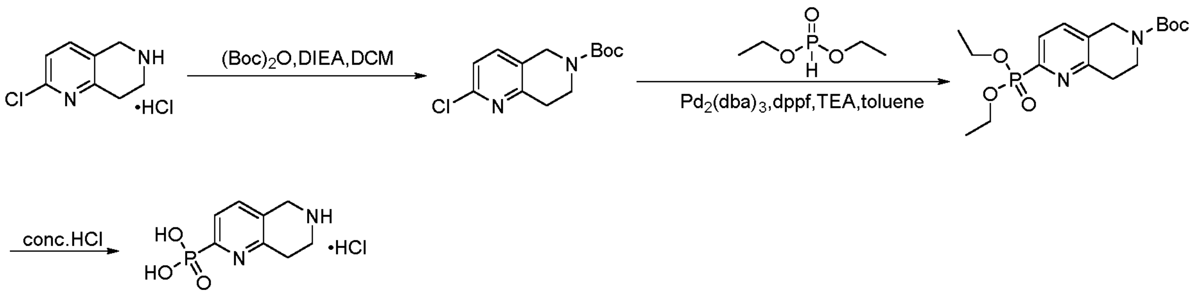


Step 1: Preparation of tert-butyl 2-chloro-7,8-dihydro-1,6-naphthyridine-6-(5H)-carboxylate

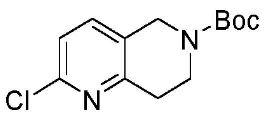

2-chloro-5,6,7,8-tetrahydro-1,6-naphthyridine hydrochloride (0.9 g) was suspended in 15 mL of dichloromethane, and *N,N-* diisopropylethylamine (1.4 g) was added, subsequently di-tert-butyl dicarbonate (1.15 g) was added, and the mixture was reacted at room temperature for 1 hour. When the TLC showed that the raw materials were completely consumed, water was added to dilute the reaction solution, and the reaction solution was extracted with dichloromethane; the organic phases were combined, dried over anhydrous sodium sulfate, filtered, and concentrated, and the resulting crude product was purified by column chromatography to obtain the target compound (1.12 g).

MS (ESI) m/z $(M+H)^+$ =269.0.

Step 2: Preparation of tert-butyl 2-(diethoxyphosphoryl)-7,8-dihydro-1,6-naphthyridine-6(5H)-carboxylate

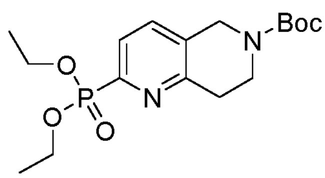

In an argon atmosphere, tert-butyl 2-chloro-7,8-dihydro-1,6-naphthyridine-6(SH)-carboxylate (100 mg) was dissolved in toluene (20 mL), and diethyl phosphite (102 mg), tris(dibenzylideneacetone)dipalladium (34 mg), 1,1'-bis(diphenylphosphino)ferrocene (41 mg), and triethylamine (75 mg) were added, and the system was reacted at 120 °C overnight. The TLC showed that the raw materials were completely consumed, ethyl acetate was added to dilute the mixture, diatomaceous earth was added to filter the mixture, , a filtrate was collected and concentrated, and the crude product was purified by preparative TLC to obtain the target compound (70 mg).

MS (ESI) m/z $(M+H)^+$=371.1.

Step 3: Preparation of (5,6,7,8-tetrahydro-1,6-naphthyridin-2-yl)phosphonic acid hydrochloride

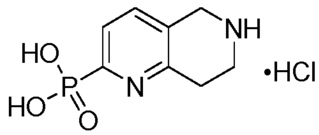

tert-butyl 2-(diethoxyphosphoryl)-7,8-dihydro-1,6-naphthyridine-6(5H)-carboxylate (70 mg) was dissolved in 5 mL of concentrated hydrochloric acid, and reacted overnight at 100 °C. After the LCMS showed that the raw materials were completely consumed, a reaction solution was concentrated, and the resulting crude product was purified by preparative HPLC to obtain the target compound (30 mg).

MS (ESI) m/z $(M+H)^+$=215.0.

$^1H$ NMR (400 MHz, $D_2O$) δ 8.35 (dd, *J* = 8.0, 2.4 Hz, 1H), 8.06 (t, *J* = 7.7 Hz, 1H), 4.59 (s, 2H), 3.67 (t, *J* = 6.0 Hz, 2H), 3.49 (t, *J* = 6.4 Hz, 2H).

**Biological tests**

**Experimental example 1**

1. Experimental purpose

The purpose is to determine the inhibitory activity of the compounds of the present disclosure on total proteases in the homogenate of the jejunal segment of the small intestine of rats.

2. Experimental materials and instruments

Experimental animals

| Strain/Grade | Sex/Week Old | Source |
|---|---|---|
| SD rats | Male, 6-7 weeks old | Beijing Vital River |

Main reagents and consumables

| Reagent Name | Supplier | Article No. |
|---|---|---|
| Protease Activity Assay Kit | Abcam | ab111750 |
| BCA Protein Concentration Assay Kit | Beyotime | P0012 |

Instruments

| Instrument Name | Manufacturer | Model |
|---|---|---|
| Multifunctional microplate reader | BMG LABTECH | PHERAstar FSX |
| Centrifuge | Eppendorf | 5424R |
| Centrifuge | Eppendorf | 5810R |
| High-throughput cryogenic grinder | Bionoon | BIONOON-96LD |

3. Experimental steps

a) Preparation of rat intestinal homogenate and protein quantification

i. Tissue sampling: The experimental animals were anesthetized and bled to death. The small intestine was folded in half from the ileocecal junction to the duodenum at the lower end of the stomach, and the jejunum in the middle with a size of about 4 cm was taken; after washing with a 0.9% sodium chloride solution, it was quickly frozen in liquid nitrogen and then transferred to -80°C for storage;
ii. Homogenization of intestinal tissue: The intestinal tissue was cut into small pieces with sterile surgical scissors on ice and transferred to a 2-mL EP tube; 4 μL of Assay Buffer (Protease Activity Assay Kit, Abcam, ab111750) was added to every 1 mg of tissue; two small steel beads were added to each EP tube, and the tissue was homogenized with a high-throughput cryogenic grinder at 3600 rpm and 4°C for 30 seconds each time, totally 2 times;
iii. The tissue homogenate was centrifuged at 12,000 g and 4°C for 10 minutes, then the homogenate supernatant (intestinal homogenate) was transferred to a new EP tube carefully, aliquoted, and stored at -80°C.
iv. The total protein of the homogenate was quantified using the BCA protein concentration assay kit according to the instructions.

b) Inhibitory activity test of compound

i. Compound stock solution: The compound was dissolved in Assay Buffer to prepare a 400 mM stock solution, which was aliquoted and stored at -20°C;
ii. 4×compound working solution: The compound stock solution was diluted with the Assay Buffer by 3-fold gradiently, with totally 10 concentration points. The wells without the compound were set as negative controls, and the wells without protease homogenate were set as blank controls;
iii. 4×intestinal protease working solution: The intestinal homogenate was diluted with the Assay Buffer to the specified concentration;
iv. Taking 200 μL of Reaction Mix solution as an example, the Reaction Mix solution was prepared according to the table below:

| Reagent | Volume (μL) |
|---|---|
| Protease Substrate Solution | 2 |
| Assay Buffer | 198 |
| Reaction Mix | 200 |

v. 10 μL of 4×compound working solution, 10 μL of 4×intestinal protease working solution, and 20 μL of Reaction Mix solution were added to a 384-well black flat bottom reaction plate, and centrifuged at 3000 rpm for 10 seconds, and then tested immediately;
vi. The fluorescence signals at FI = Ex 485 /Em 520 nm were detected continuously within 30 minutes (once every 5 minutes) using a kinetic mode.

c) Data analysis

The difference between fluorescence signals within 30 minutes was calculated and recorded as ΔRFU.
Using the log value of the compound concentration as X-axis and the difference between the fluorescence signals (ΔRFU) as Y-axis, the dose-effect curves were fitted by the four-parameter model (log(inhibitor) vs. response-(variable slope)) of the analysis software GraphPad 7, to obtain the $IC_{50}$ value of each compound on the enzyme activity.

$$\text{Fitting formula: } Y = \min + (\max - \min)/(1+10^{\wedge}((\text{LogIC50}-X)\times \text{Hillslope})).$$

The inhibitory effect of the compounds of the present disclosure on the total proteases in the rat small intestine homogenate was determined by the above test, and the measured $IC_{50}$ values were as follows.

| IC50 (mM) | |
|---|---|
| Preparative example 1 | Preparative example 2 |
| 13.4 | 17.9 |

The experimental data showed that the compounds of the present disclosure had certain inhibitory activity on total proteases in small intestines of rats.

**Experimental example 2**

1. Experimental purpose

The purpose is to determine the inhibitory activity of the compounds of the present disclosure on recombinant human proteases Trypsin, MMP9, and TACE.

2. Experimental materials and instruments

Main reagents and consumables

| Reagent | Supplier | Art. No. |
|---|---|---|
| Trypsin | Elastin Products Company, Inc. | TR127 |
| MMP9 | R&D | 911-MP-010 |
| TACE | R&D | 930-ADB |
| Trypsin substrate | Peptide Institute | 3107-V |
| MMP9 substrate | R&D | ES001 |
| TACE substrate | R&D | ES003 |
| 384-plate | Greiner | 781076 |

Instruments

| Instrument | Manufacturer | Model |
|---|---|---|
| Multifunctional microplate reader | Molecular Devices | SpectraMax M5 |

Reaction buffers

Trypsin: 100 mM Tris-HCl, 75 mM NaCl, 2.5 mM CaCl2, 10 mM Cysteine, pH 7.5
MMP9: 50 mM Tris, 10 mM $CaCl_2$, 150 mM NaCl, 0.05 % Brij-35 (w/v), pH7.5
TACE: 25 mM Tris, 2.5 μM $ZnCl_2$, 0.005 % Brij-35 (w/v), pH 9.0

3. Experimental steps

a) The compound was dissolved in a PBS or NaOH aqueous solution of equimolar volume concentration to obtain a 100 mM stock solution.
b) The compound was diluted with a PBS by 3-fold gradiently, with totally 10 concentration points.

c) 5 $\mu$L of the compound solution after the gradient dilution was added to the test plate.

d) The 4×enzyme working solutions of Trypsin, MMP9, and TACE were prepared respectively, and 5 $\mu$L of each prepared enzyme working solution was added to the test plate to make the final concentrations to be 75 nM, 0.2 $\mu$g/mL, and 0.2 $\mu$g/mL, respectively.

e) 2×substrate solution was prepared, and 10 $\mu$L of the prepared substrate solution was added to the Trypsin, MMP9, and TACE test plates respectively, to make the final concentrations to be 120, 10, and 20 $\mu$M, respectively.

f) The MMP9 and TACE were incubated at room temperature for 30 minutes or the Trypsin was incubated at 37°C for 60 minutes, and then the fluorescence values were measured using a multifunctional microplate reader.

g) Using the signals of the wells containing protein and substrate but no compound as the High Controls and the signals of the wells containing substrate but no protein and compound as the Low Controls, the inhibition rates of each test well were calculated according to the following formula:

$$\text{Inhibition rate\%}=100-100\times(\text{test well-Low Control})/(\text{High Control-Low Control})$$

h) Using the log value of concentration as the X-axis and the inhibition rat as the Y-axis, the dose-effect curves were fitted by Mode 205 of the analysis software IDBS_XLFit to obtain the $IC_{50}$ values of each compound on the enzyme activity.

$$\text{Fitting formula: fit} = (A+((B-A)/(1+((C/x)^D))));$$

A: Bottom; B: Top; C: IC50; D: Hillslope

The inhibitory effect of the compounds of the present disclosure on the above three proteases was determined by the above test, and the measured $IC_{50}$ values were as follows.

| Digestive enzyme | $IC_{50}$ (mM) | |
|---|---|---|
| | Preparative example 1 | Preparative example 2 |
| MMP9 | 6.56 | about 25 |
| TACE(ADAM17) | 5.43 | 7.38 |
| Trypsin | 9.38 | >25 |

The experimental data showed that the compounds of the present disclosure had a certain inhibitory effect on the three proteases.

**Experimental example 3**

3.1 Experimental animals: The male SPF SD rats (220 g each) were purchased from the Experimental Animal Center of Hangzhou Medical College and passed the inspection of Zhejiang Provincial Experimental Animal Quality Supervision and Inspection Station. Thirty SD rats were adaptively fed for 10 days after entering the laboratory and numbered with ear tags, and randomized into 3 groups, with 10 animals in each group.

3.2 Test samples: The active compound to be tested for test sample 1 was 5,6,7,8-tetrahydro-1,6-naphthyridine-2-carboxylic acid, and the active compound to be tested for test sample 2 was (5,6,7,8-tetrahydro-1,6-naphthyridine-2-yl)phosphonic acid. The solvent used for intragastric administration was compound polyethylene glycol electrolyte solution; the solvent used for irrigation administration was sterile saline solution.

3.3 Preparation of solvent: One bag of compound polyethylene glycol electrolyte solution (68.56 g) was dissolved in 1 L of pure water, and is further added with 44 g of glucose (containing water) to make the glucose content of 4% (w/v) in the solvent. The prepared solvent was stored at 2-8 °C and used within one week.

3.4 Preparation of intragastric solution: An amount of test sample was weighed and added with an appropriate amount of sterile ultrapure water to prepare a 20% (w/v) stock solution, then an equivalent amount of NaOH was added to adjust the pH to neutral until completely dissolved (the pH value was measured). One part of the stock solution was added to 9 parts of electrolyte solution (1:9, v:v) to prepare a 2% (w/v) intragastric solution (the pH range was measured).

3.5 Preparation of irrigation solution: An amount of test sample was weighed and added with normal saline to prepare a 1% (w/v) peritoneal irrigation solution (the pH was adjusted to neutral).

3.6 Modeling: The abdominal adhesion model was established by the file method. The animals were fasted but

water was given before operation (>12 hours). The abdomen was locally depilated after inhalation anesthesia and disinfected with iodophor solution. An incision with the size of 3 cm was made in the midline area of the abdomen of the experimental animal using a scalpel, and the cecum was taken out. The serosal layer was repeatedly rubbed with a file on the right side of the ileocecal region until there were needle-like bleeding points on the surface, forming a damaged wound of about 0.5 cm×0.5 cm. The cecum was returned back to the abdominal cavity. After irrigation administration, the abdominal cavity was closed and sutured layer by layer with surgical sutures. The experimental operations were carried out for 3 animals in each group. A sham operation group was established as a control, and the animals' abdomens were opened only without damaging the cecum.

3.7 Administration: The animals were given a single dose at 12 mL/kg by oral gavage 2 hours before the laparotomy operation. After the cecum was damaged, the animal's abdominal cavity was irrigated once at 2 mL/animal before suturing the abdomen.

| Group | Route of administration | Drug | Volume of administration |
|---|---|---|---|
| Sham operation group | Gavage + irrigation | Solvent + normal saline | 12 mL/kg, 2 mL/animal |
| Model group | Gavage + irrigation | Solvent + normal saline | 12 mL/kg, 2 mL/animal |
| Drug administration group | Gavage + irrigation | Solution of test sample 2 | 12 mL/kg, 2 mL/animal |

3.8 Scoring of adhesions: The postoperative intra-abdominal adhesions were scored according to the Phillips grading criteria, as shown in the table below.

| Grade | Area of Adhesions | Score |
|---|---|---|
| 0 | No adhesion | 0 |
| I | Adhesion area: <20% | 1 |
| II | Adhesion area: 20%-40% | 2 |
| III | Adhesion area: 40%-60% | 3 |
| IV | Adhesion area:≥60% | 4 |

3.9 Results

The animals were executed and dissected on the 7$^{th}$ day after the operation. The intestinal adhesions were scored using the Phillips grading method. The results were shown in FIG. 1 (sham operation group), FIG. 2 (model group) and FIG. 3 (test sample 2). In the sham operation group, no adhesions were observed in the abdominal cavity and cecum 7 days after modeling; in the model group, multiple and large-area intestinal adhesions were observed 7 days after modeling by cecal friction; after animals received intragastric administration with 2% solution of test sample 2 and peritoneal irrigation with 1% solution, the quantity and extent of intestinal adhesions were significantly improved in more than 80% of animals compared with the model group.

**Experimental example 4**

4.1 Experimental animals

Seventy male SPF SD rats, 220±20 g each, were purchased from Beijing Vital River Experimental Animal Technology Co., Ltd. After 2 weeks of adaptive feeding in the laboratory, the animals were numbered by ear tags and randomized into 7 groups, with 8 animals in each group.

4.2 Preparation of test samples and solvents

Test samples: The active compound to be tested for test sample 1 was 5,6,7,8-tetrahydro-1,6-naphthyridine-2-carboxylic acid hydrochloride, and the active compound to be tested for test sample 2 was (5,6,7,8-tetrahydro-1,6-naphthyridine-2-yl)phosphonic acid hydrochloride. The positive drug was tranexamic acid (TXA).

Solvent: One bag of compound polyethylene glycol electrolyte solution (68.56 g) was dissolved in 1 L of pure water, and is further added with 44 g of glucose (containing water) to make the glucose content of 4% (w/v) in the solvent. The prepared solvent was stored at 2-8 °C and used within one week.

Intragastric solution of test sample: An amount of the test sample 1 was weighed and added with the electrolyte

solution to prepare 64 mM and 21 mM solutions, then added with an appropriate amount of NaOH to adjust the pH to about 7.5, and observed; there was no precipitation. An amount of the test sample 2 was weighed to prepare a 64 mM clear solution, and is added with an appropriate amount of NaOH to adjust the pH to neutral. An amount of the positive drug tranexamic acid (TXA) was weighed and added with the electrolyte solution to prepare 64 mM and 21 mM solutions.

4.3 Grouping and administration

The animals were given a single dose at 12 mL/kg by oral gavage 2 hours before the laparotomy operation. The animals in the sham operation group and the model group were given the solvents.

| Group | Number of Animals (n) | Route of administration | Drug | Volume of administration |
|---|---|---|---|---|
| Sham operation group | 8 | Gavage | Solvent | 12 mL/kg |
| Model group | 8 | Gavage | Solvent | 12 mL/kg |
| Tranexamic acid | 8 | Gavage | 64 mM solution | 12 mL/kg |
| Tranexamic acid | 8 | Gavage | 21 mM solution | 12 mL/kg |
| Test sample 1 | 8 | Gavage | 64 mM solution | 12 mL/kg |
| Test sample 1 | 8 | Gavage | 21 mM solution | 12 mL/kg |
| Test sample 2 | 8 | Gavage | 64 mM solution | 12 mL/kg |

4.4 Modeling

The animals were fasted but water was given before the operation (>12 hours). The abdomen was locally depilated after inhalation anesthesia and disinfected with iodophor solution. An incision with the size of 3 cm was made in the midline area of the abdomen of the experimental animal using a scalpel, and the cecum was taken out. The serosal layer on each side of the ileocecal region was repeatedly rubbed with sterile gauze until there were needle-like bleeding points on the surface, forming a damaged wound inside and outside of the cecum. The cecum was returned back to the abdominal cavity. The abdominal cavity was closed, sutured layer by layer with surgical sutures, and disinfected. The entire operation should be performed aseptically. The experimental operations were carried out for 4 animals in each group. In the sham operation group, the animal' s abdomens were opened only without damaging the cecum.

4.5 Scoring of adhesions

The animals were dissected and observed 7 days after the operation. The postoperative intra-abdominal adhesions were scored according to the Phillips grading criteria, as shown in the table below.

| Grade | Area of Adhesions | Score |
|---|---|---|
| 0 | No adhesion, intact serosal surface | 0 |
| I | Adhesion area of less than 20%, a small amount of adhesions between the wound and surrounding tissue, easy to separate | 1 |
| II | Adhesion area of 20%-40%, moderate adhesions between the wound and surrounding tissues | 2 |
| III | Adhesion area of 40%-60%, more adhesions between the wound and surrounding tissues, difficult to separate | 3 |
| IV | Adhesion area of more than or equal to 60%, adhesions between the wound and surrounding tissues forming a mass, difficult to separate | 4 |

4.6 Results

The surviving animals in each group were dissected 7 days after the operation. The intestinal adhesions were scored using the Phillips grading method. The results were shown in FIG. 4. In the sham operation group, no adhesions were observed in the abdominal cavity and cecum; in the model group, multiple and large-area intestinal adhesions were observed 7 days after modeling by cecal friction. Compared with the model group, one animal died in each tranexamic acid group after the operation, the extent of intra-abdominal adhesions in the 64 mM group was significantly alleviated, and the intra-abdominal adhesions in the 21 mM group exhibited an alleviating trend. The locations and extents of intra-abdominal

adhesions were reduced significantly in rats after giving 64 mM test sample; and the locations and extents of adhesions were reduced in rats after giving 21 mM test sample 1. The locations and extents of intra-abdominal adhesions were improved to a certain extent in rats after giving 64 mM test sample 2.

The foregoing description describes the embodiments of the technical solutions of the present disclosure exemplarily. It should be appreciated that the protection scope of the present disclosure is not limited to the above embodiments. Any modification, equivalent substitution and improvement, etc. made by those skilled in the art within the spirit and principle of the present disclosure shall fall within the scope of protection as claimed by the appended claims of the application.

**Claims**

1. Use of a tetrahydronaphthyridine derivative in preparing a medicine for preventing and treating gastrointestinal tract injury-related diseases,
   the tetrahydronaphthyridine derivative being selected from 5,6,7,8-tetrahydro-1,6-naphthyridine-2-carboxylic acid, 5,6,7,8-tetrahydro-1,6-naphthyridine-2-phosphonic acid, and a hydrate, or a pharmaceutically acceptable salt, or a prodrug, or a mixture thereof.

2. The use according to claim 1, wherein the gastrointestinal tract injury-related diseases refer to adhesions.

3. The use according to claim 2, wherein the adhesions are postoperative adhesion or complications caused by the postoperative adhesions.

4. The use according to claim 3, wherein the postoperative adhesions are selected from one or more of intestinal adhesions, abdominal adhesions, intercostal adhesions, peritoneal adhesions, and intrauterine adhesions.

5. The use according to claim 3, wherein the complication caused by postoperative adhesions are selected from one or more of female infertility, postoperative intestinal obstruction, and pain.

6. The use according to any one of claims 1 to 5, wherein the pharmaceutically acceptable salt is a hydrochloride of the tetrahydronaphthyridine derivative.

7. Use of a tetrahydronaphthyridine derivative in the preparation of a medicine for inhibiting gastrointestinal protein kinase,
   the tetrahydronaphthyridine derivative being selected from 5,6,7,8-tetrahydro-1,6-naphthyridine-2-carboxylic acid, 5,6,7,8-tetrahydro-1,6-naphthyridine-2-phosphonic acid, and a hydrate, or a pharmaceutically acceptable salt, or a prodrug, or a mixture thereof.

8. The use according to claim 7, wherein the gastrointestinal protein kinase is selected from at least one of Trypsin, MMP9, and TACE.

DRAWINGS

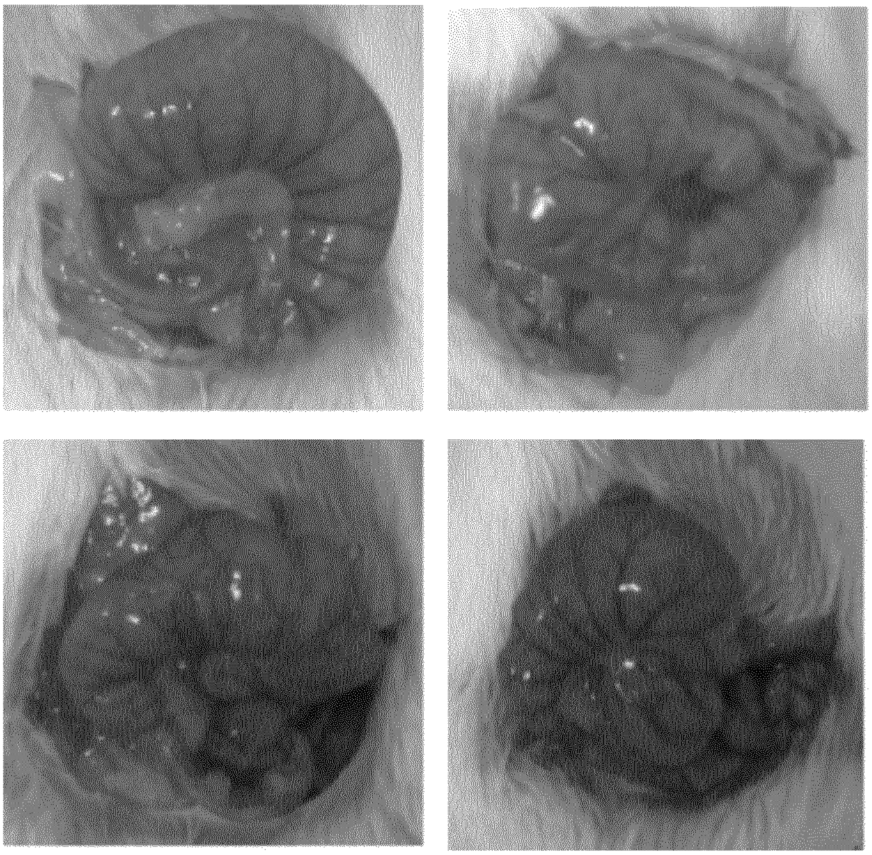

FIG. 1

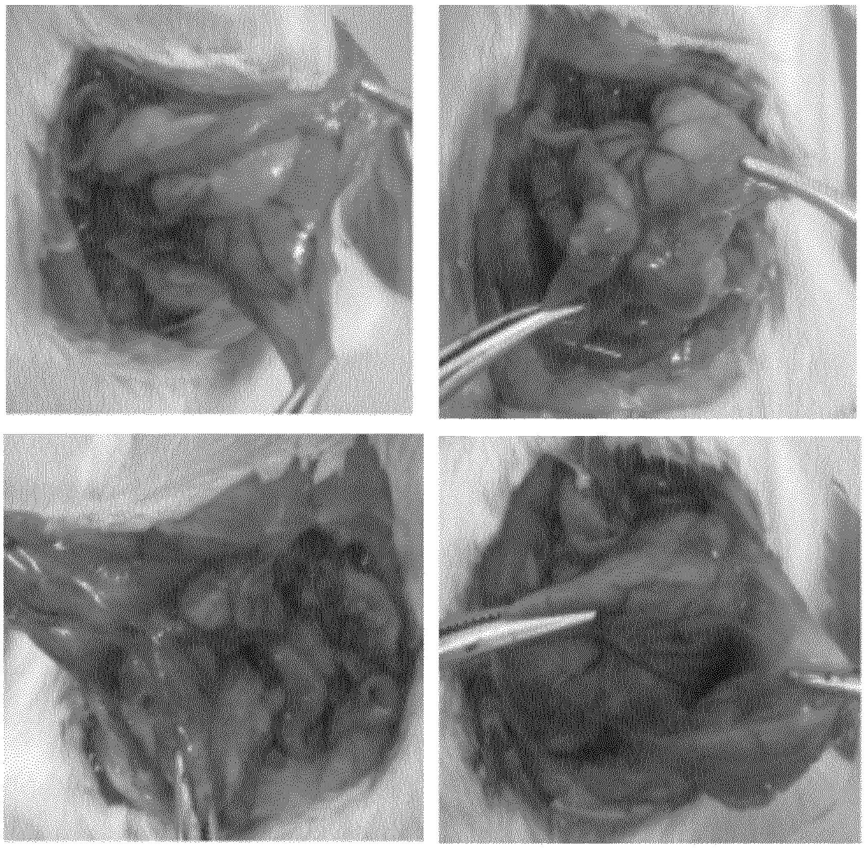

FIG. 2

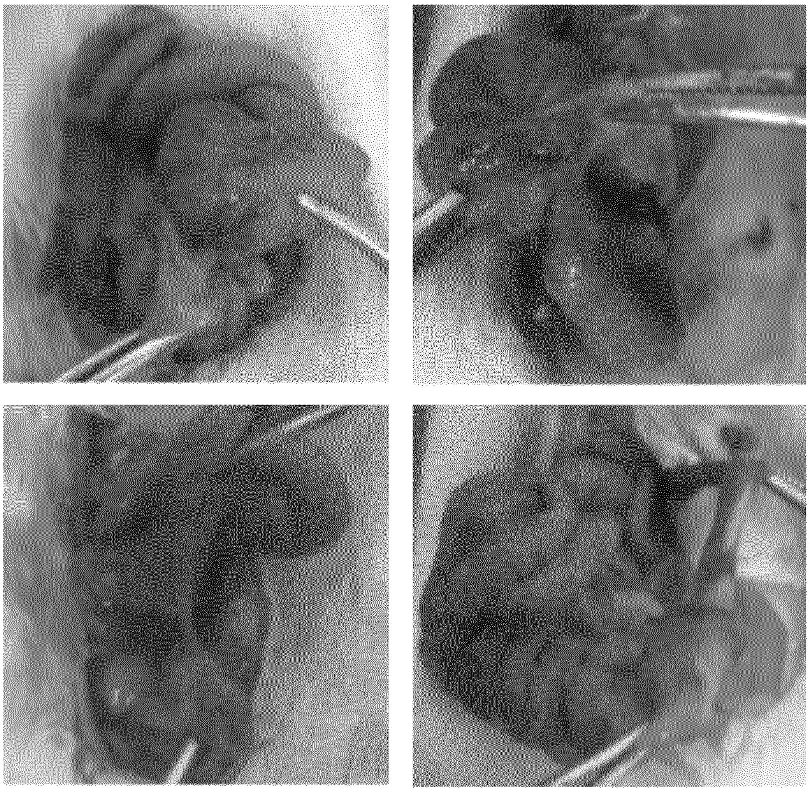

FIG. 3

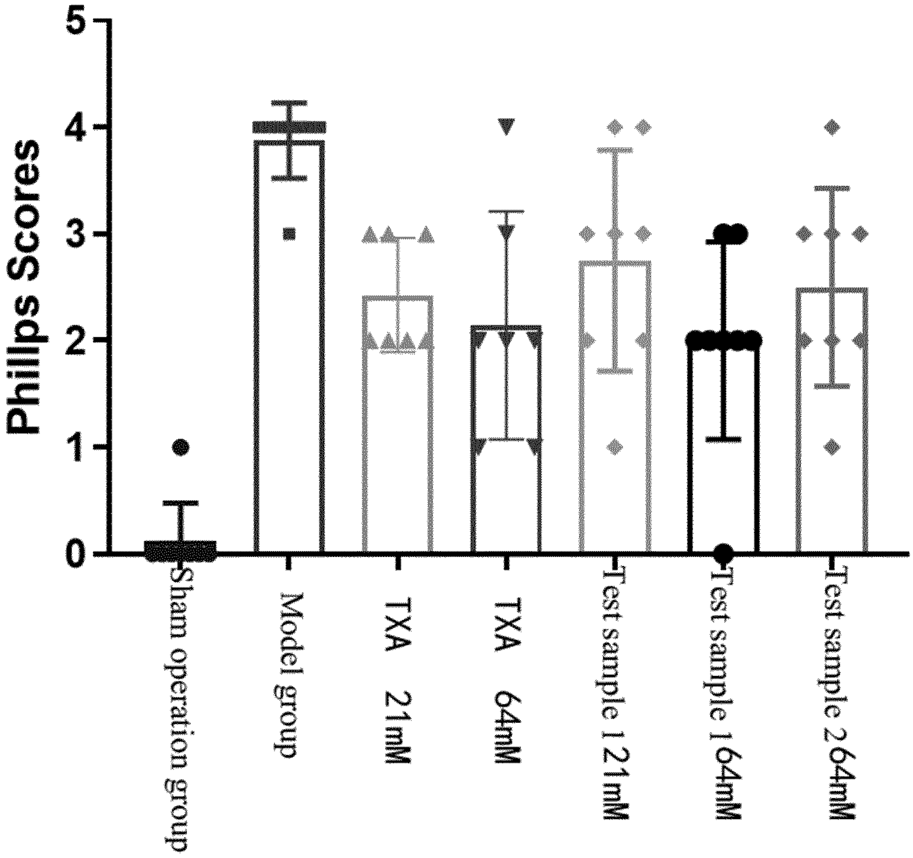
Phillps Scores
5
4
3
2
1
0
Sham operation group
Model group
TXA 21mM
TXA 64mM
Test sample 121mM
Test sample 164mM
Test sample 264mM

FIG. 4

## INTERNATIONAL SEARCH REPORT

International application No.
**PCT/CN2023/096840**

**A. CLASSIFICATION OF SUBJECT MATTER**

A61K31/4375(2006.01)i; A61P41/00(2006.01)i; A61P1/00(2006.01)i

According to International Patent Classification (IPC) or to both national classification and IPC

**B. FIELDS SEARCHED**

Minimum documentation searched (classification system followed by classification symbols)

IPC: A61K 31/-; A61P 41/-; A61P 1/-

Documentation searched other than minimum documentation to the extent that such documents are included in the fields searched

Electronic data base consulted during the international search (name of data base and, where practicable, search terms used)

CNTXT, WPABS, WPABSC, ENTXT, ENTXTC, DWPI, CNKI, REGISTRY, CAPLUS, MARPAT: 诺赛哈勃, 四氢萘啶, 肠, 胃, 粘连, 不孕, 梗阻, 疼痛, 蛋白酶, 蛋白激酶, trypsin, mmp9, TACE, adhesion, gastro+, infertility, sterility, aciesis, obstructi+, pain, 5, 6, 7, 8-四氢-1, 6-萘啶-2-甲酸结构检索, structural search of 5,6,7,8-tetrahydro-1,6-naphthyridine-2-carboxylic acid, 5, 6, 7, 8-四氢-1, 6-萘啶-2-膦酸结构检索, structural search of 5,6,7,8-tetrahydro-1,6-naphthyridine-2-phosphonic acid

**C. DOCUMENTS CONSIDERED TO BE RELEVANT**

| Category* | Citation of document, with indication, where appropriate, of the relevant passages | Relevant to claim No. |
|---|---|---|
| PA | WO 2022143911 A1 (SCINNOHUB PHARMACEUTICAL CO., LTD.) 07 July 2022 (2022-07-07)<br>abstract, and claims 1-16 | 1-8 |
| A | US 2019177301 A1 (BOARD OF REGENTS, THE UNIVERSITY OF TEXAS SYSTEM) 13 June 2019 (2019-06-13)<br>abstract, and claim 1 | 1-8 |
| A | WO 2009056556 A1 (SMITHKLINE BEECHAM CORP.) 07 May 2009 (2009-05-07)<br>abstract, and claims 1-22 | 1-8 |
| A | US 6294547 B1 (NIPPON KAYAKU CO., LTD.) 25 September 2001 (2001-09-25)<br>abstract | 1-8 |
| A | US 2008293743 A1 (ASTRAZENECA AB) 27 November 2008 (2008-11-27)<br>abstract | 1-8 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **13 September 2023** | **27 September 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2023/096840**

| Patent document cited in search report | | | Publication date (day/month/year) | Patent family member(s) | | | Publication date (day/month/year) |
|---|---|---|---|---|---|---|---|
| WO | 2022143911 | A1 | 07 July 2022 | CA | 3203896 | A1 | 07 July 2022 |
| | | | | TW | 202233199 | A | 01 September 2022 |
| US | 2019177301 | A1 | 13 June 2019 | US | 10954217 | B2 | 23 March 2021 |
| | | | | CA | 3022512 | A1 | 02 November 2017 |
| | | | | EP | 3448520 | A1 | 06 March 2019 |
| | | | | EP | 3448520 | A4 | 29 July 2020 |
| | | | | US | 2021300903 | A1 | 30 September 2021 |
| | | | | WO | 2017190109 | A1 | 02 November 2017 |
| | | | | AU | 2017257153 | A1 | 22 November 2018 |
| WO | 2009056556 | A1 | 07 May 2009 | GB | 0721419 | D0 | 12 December 2007 |
| US | 6294547 | B1 | 25 September 2001 | CA | 2295309 | A1 | 07 January 1999 |
| | | | | EP | 0997462 | A1 | 03 May 2000 |
| | | | | EP | 0997462 | A4 | 04 July 2001 |
| | | | | EP | 0997462 | B1 | 22 December 2004 |
| | | | | WO | 9900388 | A1 | 07 January 1999 |
| | | | | DE | 69828284 | D1 | 27 January 2005 |
| | | | | DE | 69828284 | T2 | 08 December 2005 |
| | | | | PL | 337672 | A1 | 28 August 2000 |
| | | | | AT | 285410 | T | 15 January 2005 |
| | | | | AU | 7935298 | A | 19 January 1999 |
| | | | | AU | 729995 | B2 | 22 February 2001 |
| | | | | KR | 20010014262 | A | 26 February 2001 |
| US | 2008293743 | A1 | 27 November 2008 | UA | 90285 | C2 | 26 April 2010 |
| | | | | MX | 2007007023 | A | 11 September 2007 |
| | | | | AU | 2005317286 | A1 | 22 June 2006 |
| | | | | AU | 2005317286 | B2 | 05 February 2009 |
| | | | | US | 7655664 | B2 | 02 February 2010 |
| | | | | SE | 0403085 | D0 | 17 December 2004 |
| | | | | WO | 2006065215 | A1 | 22 June 2006 |
| | | | | CA | 2590845 | A1 | 22 June 2006 |
| | | | | NO | 20073572 | L | 12 September 2007 |
| | | | | JP | 2008524210 | A | 10 July 2008 |
| | | | | NZ | 555831 | A | 28 January 2011 |
| | | | | US | 2010256166 | A1 | 07 October 2010 |
| | | | | AT | 545642 | T | 15 March 2012 |
| | | | | BRPI | 0517035 | A | 30 September 2008 |
| | | | | SA | 05260409 | B1 | 14 March 2009 |
| | | | | ES | 2380670 | T3 | 17 May 2012 |
| | | | | TW | 200635917 | A | 16 October 2006 |
| | | | | EP | 1831199 | A1 | 12 September 2007 |
| | | | | EP | 1831199 | B1 | 15 February 2012 |
| | | | | IL | 183599 | A0 | 20 September 2007 |
| | | | | IL | 183599 | A | 29 February 2012 |
| | | | | RU | 2007126746 | A | 27 January 2009 |
| | | | | RU | 2376301 | C2 | 20 December 2009 |
| | | | | ZA | 200705075 | B | 31 December 2008 |
| | | | | MY | 147770 | A | 31 January 2013 |
| | | | | KR | 20070090924 | A | 06 September 2007 |
| | | | | AR | 051795 | A1 | 07 February 2007 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202210596510
- CN 202211286168